# FASCICULE DE BREVET EUROPEEN

(11) **EP 3 297 692 B1**
(45) Date de publication et mention de la délivrance du brevet: **05.01.2022**
(21) Numéro de dépôt: 16732691.7
(22) Date de dépôt: 20.05.2016
(51) Int. Cl.: A61L 24/06, A61L 24/00

(54) **COLLES CHIRURGICALES**
CHIRURGISCHER KLEBSTOFF
SURGICAL ADHESIVES

(30) Priorité: 21.05.2015 FR 1554583
(43) Date de publication de la demande: 28.03.2018
(73) Titulaire: Cohesives, 21000 Dijon (FR); Université de Pau et des Pays de l'Adour, 64000 Pau (FR); Université de Bordeaux, 33000 Bordeaux (FR); Institut Polytechnique de Bordeaux, 33400 Talence (FR); Centre National de la Recherche Scientifique, 75016 Paris (FR)
(72) Inventeur: PERRIN, Bertrand, 21000 Dijon (FR); DERAIL, Christophe, 64170 Cescau (FR); BADIE, Laetitia, 65420 Ibos (FR); PAPON, Eric, 33350 Saint Magne de Castillon (FR)
(74) Mandataire: Oudin, Stéphane
(86) Numéro de dépôt international: PCT/FR2016/051211
(87) Numéro de publication internationale: WO 2016/185153

(56) Documents cités:
- EP-A1- 1 994 886
- WO-A2-2012/088059
- US-A- 4 181 752
- US-A1- 2007 134 333
- L Badie ET AL: "Photo-polymerized adhesives for biological tissues", , 1 janvier 2015 (2015-01-01), XP055255946, Extrait de l'Internet: URL:http://www.radtech2015.com/downloads/7 1-Badie.pdf [extrait le 2016-03-07]

## Description

### Domaine technique

La présente invention appartient au domaine des colles chirurgicales, plus particulièrement la présente invention concerne des compositions destinées à être utilisées dans un procédé pour l'adhésion de tissus biologiques entre eux, pour l'adhésion d'un matériau à un tissu biologique, pour l'adhésion d'une colle ou d'une substance à la surface d'un tissu biologique, pour obturer un orifice (hémostase, aérostase) dans un tissu biologique, pour renforcer un tissu biologique et/ou, pour fixer et stabiliser un tissu biologique.

### Technique antérieure

Un certain nombre de techniques chirurgicales mettent en œuvre des colles chirurgicales. Ces dernières sont principalement utilisées pour aider à obtenir une hémostase chirurgicale. Toutefois, l'efficacité des colles chirurgicales dans cette indication est controversée et d'autres utilisations comme pour l'aérostase ne montrent pas de meilleurs résultats.

Par ailleurs, les colles chirurgicales ont de très faibles propriétés adhésives et ne peuvent donc pas être utilisées en tant qu'adhésif ni comme suture chirurgicale. L'application des colles chirurgicales se fait la plupart du temps directement sur le tissu, sans préparation de la surface de collage. La pénétration dans les tissus est faible ou inexistante ce qui entraine un collage de mauvaise qualité. Les déposants ont constaté que les colles actuelles ne collent pas et ne pénètrent pas dans les tissus. En conséquence, ils ont mis au point une colle apte à pénétrer en profondeur dans la superficie du tissu biologique afin de réaliser un ancrage de la colle dans le tissu.

Par exemple, on connaît du document EP1994886A1, une colle chirurgicale comprenant des monomères polymérisables de la famille des cyanoacrylates. La polymérisation de ces derniers est déclenchée par l'humidité du tissu biologique dès que le contact se fait entre la colle chirurgicale et celui-ci. En conséquence, malgré la faible viscosité de cette colle chirurgicale, la polymérisation des monomères de cyanoacrylate a lieu à la surface du tissu biologique. Ainsi, les monomères de cyanoacrylates ne peuvent pas pénétrer dans le tissu biologique. Les monomères de cyanoacrylate ne peuvent pas s'ancrer dans le tissu ce qui explique la faible résistance mécanique et la faible efficacité clinique des colles chirurgicales à base de cyanoacrylate.

US2007/0134333 décrit des adhésifs de type acrylates polymérisables sous l'effet des UV en présence d'un photoamorceur. Toutefois, les compositions décrites comprennent des polymères et non pas des monomères ou oligomères.

Ainsi la présente invention se propose de fournir un nouveau type de colles chirurgicales. Les compositions et le procédé selon l'invention permettent d'obtenir un collage efficace et résistant. La rupture du collage se fait par la propagation d'une fissure dans le tissu collé ou dans le joint de colle et non à l'interface colle-tissu. Le collage est applicable à tout type de tissus biologiques (tissus mous, os). Un tel collage permet par ailleurs d'obtenir une hémostase ou une aérostase efficace. Il permet également de remplacer la suture chirurgicale par un collage.

### Résumé de l'invention

Le principe de l'invention consiste à laisser pénétrer un monomère polymérisable dans le tissu biologique afin de permettre un ancrage du polymère dans le tissu, ce qui renforce les propriétés d'adhésion à la surface du tissu.

Ainsi, la présente invention concerne une composition destinée à être utilisée dans un procédé pour l'adhésion de tissus biologiques entre eux, pour l'adhésion d'un matériau à un tissu biologique, pour l'adhésion d'une colle ou d'une substance à la surface d'un tissu biologique, pour obturer un orifice dans un tissu biologique, pour renforcer un tissu biologique et/ou pour fixer et stabiliser un tissu biologique remarquable en ce qu'elle comprend un photo-amorceur, un monomère d'acrylate ou de méthacrylate ou un oligomère d'acrylate ou de méthacrylate polymérisable sous l'effet d'un rayonnement ultra-violet (UV), et ne comprend pas de monomères polymérisables de la famille des cyanoacrylates , et en ce que sa viscosité est inférieure à 10 mPa.s à 20°C.

La viscosité de la composition peut notamment être mesurée par un viscosimètre à chute de bille selon la norme DIN53015.

En effet, les déposants ont pu mettre en évidence que la viscosité de ladite composition permettait d'obtenir une pénétration importante dans les tissus biologiques et une adhésion optimale.

Dans le cadre de la présente invention le terme « monomère polymérisable » entend désigner un monomère dont la polymérisation peut être initiée sous l'effet d'un rayonnement ultra-violet (UV). Ce mode d'initiation de la polymérisation permet d'attendre que la composition de monomères ait pénétré dans les tissus avant de déclencher la polymérisation. Préférentiellement, la polymérisation de la composition selon l'invention ne peut être initiée que par les rayonnements ultra-violet à l'exclusion de tout autre mode d'initiation. En particulier, l'initiation de la polymérisation des monomères polymérisables consiste en l'irradiation par rayonnements UV. De manière préférée, ledit rayonnement UV a une longueur d'onde comprise entre de 150 nm à 280 nm, encore plus préférentiellement entre 170 nm à 260 nm et tout à fait préférentiellement entre 190 nm et 240 nm.

Selon un autre mode de réalisation préféré ledit rayonnement UV a une longueur d'onde comprise entre de 200 nm à 400 nm, encore plus préférentiellement entre 300 nm à 400 nm et tout à fait préférentiellement entre 350 nm et 400 nm.

Le polymère obtenu après polymérisation du monomère est préférentiellement un polymère biocompatible.

Pour ces raisons, la composition selon l'invention ne comprend pas de monomères polymérisables dont la polymérisation peut être initiée au seul contact de molécules d'eau. On évite ainsi la polymérisation instantanée de la composition selon l'invention au contact des tissus.

Pour ces mêmes raisons, la composition selon l'invention ne comprend pas de monomères polymérisables de la famille des cyanoacrylates connues pour polymériser rapidement au contact de l'eau et/ou de l'humidité ambiante.

Préférentiellement, le monomère polymérisable est uniquement polymérisable par irradiation par rayonnements UV.

Selon un mode de réalisation préféré, ladite viscosité est inférieure à 6 mPa.s à 20°C.

Selon un mode de réalisation encore plus préféré, ladite viscosité est inférieure à 4 mPa.s à 20°C.

Selon un mode de réalisation tout à fait préféré, ladite viscosité est inférieure à 2 mPa.s à 20°C et plus particulièrement entre 1 et 2 mPa.s à 20°C.

Selon un mode de réalisation préféré, la composition selon l'invention n'est pas un hydrogel.

Selon un mode de réalisation préféré, ledit monomère comprend une fonction polaire.

Dans le cadre de la présente invention, le terme « fonction polaire » fait référence à un groupe d'atomes dans lequel les électrons sont répartis de façon asymétrique, permettant ainsi à cette fonction polaire de prendre part à des interactions électrostatiques. Ladite fonction polaire peut notamment être choisie dans le groupe comprenant les fonctions hydroxyle, amide, carboxyle, amino, carbonate, carbamate, sulfonamide, sulfonique, phosphonique, méthoxyéthyle, méthoxyéthoxyéthyle, hydroxyéthyle et hydroxyéthoxyéthyle.

Selon un mode de réalisation préféré, ledit monomère d'acrylate est choisi dans le groupe comprenant les mono-, di-, tri-, tetra- et penta-acrylate ou méthacrylate, et leurs mélanges.

Selon un mode de réalisation préféré, ledit monomère d'acrylate est choisi dans le groupe comprenant l'acide acrylique, le méthacrylate de méthyle; le méthacrylate de diméthylaminoéthyle ; l'acrylate d'éthyle; le méthacrylate de cyclohexyle; le méthacrylate de 2-hydroxyethyle; l'acrylate de 3-hydroxypropyle; l'acrylate d'alpha-bromoéthyle; l'acrylate d'alpha-chloroéthyle; le méthacrylate de chlorométhyle; le méthacrylate de 2-bromoéthyle; le méthacrylate de 2-naphtyle; l'acrylate de paratolyle; le méthacrylate de parachlorophényle; l'acrylate de metabromophényle; l'acrylate de 2,4,6-tribromophényle; le méthacrylate de parachlorobenzyle; le méthacrylate de metaméthoxybenzyle; l'acrylate de paraéthylbenzyle; le di méthacrylate de 1,6-hexanediol; le di acrylate de neopentylglycol; le di méthacrylate de thiodiéthylène-glycol; le di acrylate de bisphénol A éthoxyle; le di méthacrylate de bisphénol A éthoxyle; le triacrylate de pentaérythritol; le triacrylate de glycéryle; le pentaacrylate de dipentaérythritol; le triacrylate de triméthylolpropane; le tri méthacrylate de l'isocyanurate de tris (2-hydroxyéthyle); le triacrylate de triméthylolpropane polyoxyéthylène; un acrylate d'uréthane; un methacrylate d'uréthane; le sulfure de bis(4-methacryloylthiophényle); l'acrylate de tert-butyle ; un éthylèneglycol ou un polyéthylèneglycol choisi dans le groupe constitué par l'acrylate, le methacrylate; le diacrylate, le dimethacrylate, et leurs mélanges.

Selon un mode de réalisation tout à fait préféré, ledit monomère d'acrylate est choisi dans le groupe l'Hydroxyéthyl)methacrylate, l'acide acrylique, l'Hydroxy(propyl)methacrylate, l'acrylate de tert-butyle, le méthacrylate de diméthylaminoéthyle et leurs mélanges.

Selon un mode de réalisation tout à fait préféré, ledit monomère d'acrylate est choisi dans le groupe comprenant l'acide acrylique, l'(Hydroxyéthyl)methacrylate, l'(Hydroxypropyl)methacrylate et leurs mélanges.

Selon un autre mode de réalisation tout à fait préféré, ledit monomère d'acrylate est choisi dans le groupe comprenant l'acide acrylique, l'acrylate de tert-butyle et leurs mélanges.

Selon un autre mode de réalisation tout à fait préféré, ledit monomère d'acrylate est choisi dans le groupe comprenant l'acide acrylique, le méthacrylate de diméthylaminoéthyle et leurs mélanges.

Selon un mode de réalisation préféré, ledit monomère a une masse molaire comprise entre 50 et 300g.mol⁻¹.

Selon un mode de réalisation préféré, ledit monomère a une concentration comprise entre 90 et 100% en masse par rapport à la masse totale de la composition.

Selon un mode de réalisation préféré, ladite composition comprend en outre un agent de réticulation.

L'homme du métier est apte à choisir l'agent de réticulation le plus adapté en fonction du monomère utilisé.

Selon un mode de réalisation préféré, ledit agent de réticulation comprend une fonction acrylate.

Selon un mode de réalisation préféré, ledit agent de réticulation est choisi dans le groupe comprenant les acrylates multi-fonctionnels comprenant notamment le 1,6-hexanediol di acrylate, le triméthylolpropane tri acrylate, le 1,2-éthylène glycol di acrylate, le pentaérythritol tétracrylate et les mélanges de ceux-ci.

Selon un autre mode de réalisation préféré, ledit agent de réticulation est choisi dans le groupe comprenant les acrylates multi-fonctionnels comprenant notamment l'hexanediol diméthylacrylate (HDDMA), l'éthylène glycol diméthylacrylate (EGDMA), le butanediol diacrylate (BDDA), , le poly(éthylène glycol) diacrylate (PEGDA) et les mélanges de ceux-ci.

Selon un mode de réalisation préféré, ledit agent de réticulation est présent à une concentration comprise entre 1% et 5% en masse, encore plus préférentiellement entre 1 et 3 % en masse, encore plus préférentiellement entre 1 et 2% en masse par rapport à la masse totale de la composition.

Selon un mode de réalisation préféré, ledit agent de réticulation est présent à une concentration comprise entre 0.1 et 3% en masse, encore plus préférentiellement entre 0.1 et 0.5 % en masse, encore plus préférentiellement entre 0.1 et 0.3% en masse et tout à fait préférentiellement à une concentration de 0.2% en masse par rapport à la masse totale de la composition.
La composition selon l'invention comprend un photo-amorceur. L'homme du métier choisira en fonction du spectre d'émission de la lampe utilisée le photoamorceur le plus adapté.

Le photoamorceur peut être choisi parmi : 2,2-diméthoxyphényl-2-acétophénone (DMPA) camphorquinone ou 4,4'-bis(diethylamino)benzophenone, cette liste étant non limitative.

Avantageusement le photoamorceur est utilisé à une concentration comprise entre 0,2 et 1%, préférentiellement entre 0.2 et 0.3% en masse.

Selon un mode de réalisation préféré, ledit photo-amorceur est le DMPA.

Selon un mode de réalisation de l'invention, ladite composition comprend un solvant et encore plus préférentiellement ledit solvant est de l'eau. Selon un autre mode de réalisation préféré, ledit solvant est un alcool et tout à fait préférentiellement de l'éthanol.

Selon un autre mode de réalisation préféré, ladite composition est dépourvue de solvant.

Dans le cadre de la présente invention, le terme « comprend » entend signifier que la composition selon l'invention inclut les éléments cités. De manière préférée, la présente invention concerne des compositions comprenant uniquement les éléments cités à l'exclusion de tout autre.

Les caractéristiques du rayonnement UV mis en œuvre, notamment sa puissance et sa longueur d'onde, sont adaptées aux constituants de la composition, notamment à la nature du monomère polymérisable et à sa concentration dans la composition.

Dans le cadre de la présente invention, le terme « source de rayonnement UV » fait référence à tout moyen artificiel apte à produire un rayonnement UV et plus particulièrement un rayonnement de longueur d'onde comprise entre 150 et 280 nm encore plus préférentiellement entre 170 nm à 260 nm et tout à fait préférentiellement entre 190 nm et 240 nm. De manière préférée ledit rayonnement UV est de puissance comprise entre 0.5 W et 200W et tout à fait préférentiellement entre 100 et 200W.

Selon un autre mode de réalisation préféré le terme « source de rayonnement UV » fait référence à tout moyen artificiel apte à produire un rayonnement UV d'une longueur d'onde comprise entre de 200 nm à 400 nm, encore plus préférentiellement entre 300 nm à 400 nm et tout à fait préférentiellement entre 350 nm et 400 nm.

De manière tout à fait préférée, ledit rayonnement UV est de longueur d'onde comprise entre 150 et 280 nm et de puissance comprise entre 100 et 200W.

### Description des modes de réalisation

### Matériels et méthodes

### Test de pelage

Des solutions d'acide acrylique, (Hydroxyéthyl)methacrylate/acide acrylique, (Hydroxypropyl)methacrylate/acide acrylique, acide acrylique/acrylate de tert-butyle/agents de réticulation, méthacrylate/acide acrylique/(Hydroxyéthyl)methacrylate/agents de réticulation, ou acide acrylique/ méthacrylate de diméthylaminoéthyle/agents de réticulation de viscosité et de concentrations variables ont été déposées sur des échantillons de péricardes bovins. Cette étape est réalisée à 20°C. Lesdits échantillons de péricarde ont été soumis à un rayonnement UV de 150 W, pendant une durée de 5 min., afin de déclencher la polymérisation des monomères. La source de rayonnement a été placée à 10 cm du péricarde.

Lesdits échantillons de péricarde ont été ensuite recouverts d'une bande de toile de verre, cette dernière a ensuite reçu une solution de monomères identique à celle utilisée à l'étape précédente.

Les échantillons de péricarde ont été soumis à un rayonnement UV dans des conditions identiques à celles de l'étape précédente.

Un test de pelage a ensuite été effectué par traction à 180° sur la bande de fibre de verre dans un four régulé à 37°C. Le temps de repos de la bande installée entre les mors de la machine de traction est d'une minute, la température au sein de l'échantillon est, au moment du lancement du test, de 30°C + ou - 4 °C.

### Observation au Microscope électronique à balayage environnemental

Une solution d'acide acrylique a été déposée sur des échantillons de péricarde. Lesdits échantillons de péricarde ont été soumis à un rayonnement UV de 150 W, pendant 5 min., afin de déclencher la polymérisation des monomères. La source de rayonnement a été placée à 10 cm du péricarde.

Les échantillons de péricarde ont ensuite été coupés transversalement et observés par microscopie électronique à balayage.

### Résultats

### Test de pelage

Les résultats obtenus sont présentés dans le tableau ci-dessous.

Sur tous les essais effectués, on observe, quel que soit l'adhésif utilisé, un taux d'environ 70% de rupture dans le tissu ou la bande de fibres de verre et 30% dans l'adhésif. Quand la rupture a lieu dans l'adhésif, la force nécessaire pour détruire l'assemblage est équivalente à celle obtenue pour une rupture dans la fibre de verre.

On observe que la résistance à la rupture (i.e. la résistance du collage) croît inversement à la viscosité, de la composition selon l'invention, utilisée.

**Tableau 1**

| Composition utilisée (toutes les compositions comprennent 0.25% en masse de DMPA) | Viscosité [mPa.s] | Résistance à la rupture F/b [N/m] dans le péricarde |
|---|---|---|
| 100% acide acrylique | 1 | 300 |
| 25%HEMA 75%AA | 3 | 300 |
| 50%HEMA 50%AA | 4 | 250 |
| 75%HEMA 25%AA | 6 | 200 |
| 25%HPMA 75%AA | 3 | 400 |
| 50%HPMA 50%AA | 4.5 | 300 |
| 75%HPMA 25%AA | 6.5 | 100 |
| 65% AA 35% tBuAC 2%HDDMA | 1.23 | 400 |
| 50% AA 50% tBuAC 2%HDDMA | 1.23 | 190 |
| 50% AA 25% HEMA 25% MA 2%HDDMA | 1.81 | 190 |
| 90% AA 10% DMAEMA 2%HDDMA | 9.6 | 322 |
| 65% AA 35% tBuAC 2%EGDMA | 1.18 | 343 |
| 65% AA 35% tBuAC 2%BDDA | 1.27 | 286 |
| | | |
| 65% AA 35% tBuAC 2%PEGDA | 1.5 | 382 |

### Observation au Microscope électronique à balayage

On observe la présence du polymère formé infiltré dans la surface du tissu sur une profondeur de 50µm. On observe par ailleurs que le polymère formé a pénétré dans les espaces entre les fibres de collagène des tissus.

Cette observation indique la capacité des compositions selon l'invention à pénétrer dans les tissus ce qui explique la parfaite adhésion obtenue.

| | | |
|---|---|---|
| 50% AA 25% HEMA 25% MA 2%HDDMA | 1.81 | 190 |
| 90% AA 10% DMAEMA 2%HDDMA | 9.6 | 322 |
| 65% AA 35% tBuAC 2%EGDMA | 1.18 | 343 |
| 65% AA 35% tBuAC 2%BDDA | 1.27 | 286 |
| 65% AA 35% tBuAC 2%PEGDA | 1.5 | 382 |

### Observation au Microscope électronique à balayage

On observe la présence du polymère formé infiltré dans la surface du tissu sur une profondeur de 50pm. On observe par ailleurs que le polymère formé a pénétré dans les espaces entre les fibres de collagène des tissus.

Cette observation indique la capacité des compositions selon l'invention à pénétrer dans les tissus ce qui explique la parfaite adhésion obtenue.

## Revendications

1. Composition, destinée à être utilisée dans un procédé pour l'adhésion de tissus biologiques entre eux, pour l'adhésion d'un matériau à un tissu biologique, pour l'adhésion d'une colle ou d'une substance à la surface d'un tissu biologique, pour obturer un orifice dans un tissu biologique, pour renforcer un tissu biologique et/ou pour fixer et stabiliser un tissu biologique **caractérisée en ce qu'**elle comprend un photo-amorceur, un monomère d'acrylate ou de méthacrylate ou un oligomère d'acrylate ou de méthacrylate polymérisable sous l'effet d'un rayonnement ultra-violet (UV), et ne comprend pas de monomères polymérisables de la famille des cyanoacrylates , et **en ce que** sa viscosité mesurée par un viscosimètre à chute de bille selon la norme DIN53015 est inférieure à 10 mPa.s à 20°C.

2. Composition selon la revendication précédente, **caractérisée en ce que** ledit rayonnement UV a une longueur d'onde comprise entre de 150 nm à 280 nm, encore plus préférentiellement entre 170 nm à 260 nm et tout à fait préférentiellement entre 190 nm et 240 nm.

3. Composition selon l'une des revendications précédentes, **caractérisée en ce que** le monomère polymérisable est uniquement polymérisable par irradiation par rayonnements UV.

4. Composition selon l'une des revendications précédentes, **caractérisée en ce qu'elle** ne comprend pas de monomères polymérisables dont la polymérisation peut être initiée au seul contact de molécules d'eau.

5. Composition selon l'une des revendications précédentes, **caractérisée en ce que** ledit monomère comprend une fonction polaire.

6. Composition selon l'une des revendications précédentes, **caractérisée en ce que** ledit monomère a une masse molaire comprise entre 50 et 300g.mol⁻¹.

7. Composition selon l'une des revendications précédentes **caractérisée en ce qu'**elle est dépourvue de solvant.

8. Composition selon l'une des revendications précédentes **caractérisée en ce qu'**elle comprend en outre un agent de réticulation.

9. Composition selon la revendication précédente **caractérisée en ce que** ledit agent de réticulation comprend une fonction acrylate.

## Patentansprüche

1. Zusammensetzung, die zur Verwendung in einem Verfahren zur Adhäsion von biologischen Geweben untereinander vorgesehen ist, zur Adhäsion eines Materials an ein biologisches Gewebe, zur Adhäsion eines Klebstoffs oder einer Substanz an die Oberfläche eines biologischen Gewebes, zum Verstopfen einer Öffnung in einem biologischen Gewebe, zum Verstärken eines biologischen Gewebes und/oder zum Fixieren und Stabilisieren eines biologischen Gewebes, **dadurch gekennzeichnet, dass** sie einen Fotoinitiator, ein Acrylat- oder Methacrylatmonomer oder ein Acrylat- oder Methacrylatoligomer, das unter Einwirkung einer Ultraviolettstrahlung (UV-Strahlung) polymerisierbar ist, umfasst und keine polymerisierbaren Monomere der Familie der Cyanoacrylate umfasst und dass seine Viskosität, die durch ein Kugelfall-Viskosimeter gemäß dem Standard DIN53015 gemessen wird, bei 20 °C weniger als 10 mPa.s beträgt.

2. Zusammensetzung nach dem vorhergehenden Anspruch, **dadurch gekennzeichnet, dass** die UV-Strahlung eine Wellenlänge aufweist, die zwischen 150 nm und 280 nm, noch mehr bevorzugt zwischen 170 nm und 260 nm und am meisten bevorzugt zwischen 190 nm und 240 nm liegt.

3. Zusammensetzung nach einem der vorhergehenden Ansprüche, **dadurch gekennzeichnet, dass** das polymerisierbare Monomer lediglich durch Bestrahlung mit UV-Strahlen polymerisierbar ist.

4. Zusammensetzung nach einem der vorhergehenden Ansprüche, **dadurch gekennzeichnet, dass** sie keine polymerisierbaren Monomere umfasst, deren Polymerisation durch alleinigen Kontakt mit Wassermolekülen initiiert wird.

5. Zusammensetzung nach einem der vorhergehenden Ansprüche, **dadurch gekennzeichnet, dass** das Monomer eine polare Funktion umfasst.

6. Zusammensetzung nach einem der vorhergehenden Ansprüche, **dadurch gekennzeichnet, dass** das Monomer eine Molmasse aufweist, die zwischen 50 und 300 g.mol⁻¹ liegt.

7. Zusammensetzung nach einem der vorhergehenden Ansprüche, **dadurch gekennzeichnet, dass** sie frei von Lösungsmittel ist.

8. Zusammensetzung nach einem der vorhergehenden Ansprüche, **dadurch gekennzeichnet, dass** sie weiterhin ein Vernetzungsmittel umfasst.

9. Zusammensetzung nach dem vorhergehenden Anspruch, **dadurch gekennzeichnet, dass** das Vernetzungsmittel eine Acrylatfunktion umfasst.

## Claims

1. Composition, intended to be used in a method for the adhesion of biological tissues to one another, for the adhesion of a material to a biological tissue, for the adhesion of an adhesive or of a substance to the surface of a biological tissue, for sealing an orifice in a biological tissue, for reinforcing a biological tissue and/or for fixing and stabilising a biological tissue, **characterised in that** it comprises a photoinitiator, an acrylate or methacrylate monomer or an acrylate or methacrylate oligomer polymerisable under the effect of ultra-violet (UV) radiation, and does not comprise polymerisable monomers of the cyanoacrylate family, and **in that** its viscosity, measured by a falling-ball viscometer according to the DIN53015 standard, is less than 10 mPa.s at 20°C.

2. Composition according to the preceding claim, **characterised in that** said UV ray has a wavelength comprised between 150 nm and 280 nm, still more preferably between 170 nm and 260 nm and absolutely preferably between 190 nm and 240 nm.

3. Composition according to one of the preceding claims, **characterised in that** the polymerisable monomer is only polymerisable by irradiation by UV rays.

4. Composition according to one of the preceding claims, **characterised in that it** does not comprise polymerisable monomers the polymerisation of which can be initiated just by the contact of water molecules.

5. Composition according to one of the preceding claims, **characterised in that** said monomer comprises a polar function.

6. Composition according to one of the preceding claims, **characterised in that** said monomer has a molar mass comprised between 50 and 300 g.mol⁻¹.

7. Composition according to one of the preceding claims, **characterised in that** it has no solvent.

8. Composition according to one of the preceding claims, **characterised in that** it further comprises a cross-linking agent.

9. Composition according to the preceding claim, **characterised in that** said cross-linking agent comprises an acrylate function.
